# FASCICULE DE BREVET EUROPEEN

(11) **EP 3 784 345 B1**
(45) Date de publication et mention de la délivrance du brevet: **21.06.2023**
(21) Numéro de dépôt: 19730185.6
(22) Date de dépôt: 26.04.2019
(51) Int. Cl.: A61N 5/06, D06N 3/00

(54) **PIÈCE COMPOSITE COMPRENANT UN MATÉRIAU CAPABLE DE RENVOYER DES INFRAROUGES LOINTAINS**
VERBUNDTEIL AUS EINEM MATERIAL, DAS FERNINFRAROTSTRAHLUNG REFLEKTIEREN KANN
COMPOSITE PART COMPRISING A MATERIAL THAT CAN REFLECT FAR INFRARED RADIATION

(30) Priorité: 26.04.2018 FR 1800448
(43) Date de publication de la demande: 03.03.2021
(73) Titulaire: Tortel Industries, 26220 Dieulefit (FR)
(72) Inventeur: Tortel, Arnaud Emmanuel André, 26220 Dieulefit (FR)
(74) Mandataire: Germain Maureau
(86) Numéro de dépôt international: PCT/FR2019/050993
(87) Numéro de publication internationale: WO 2019/207266

(56) Documents cités:
- WO-A1-2015/171467
- WO-A1-2017/048786
- CN-A- 106 418 794
- FR-A1- 2 948 695
- FR-A1- 3 046 936

## Description

L'invention concerne une pièce composite comprenant une surface destinée à être en contact direct avec la peau d'un individu vivant, pour procurer à ce dernier des effets sanitaires et/ou cosmétiques et/ou médicaux bénéfiques. La pièce composite intégrant un matériau susceptible de stocker et d'émettre au moins un rayonnement dans la gamme des infrarouges lointains. Un tel matériau est communément appelé céramique. Il s'agit d'un matériau incorporant au moins un oxyde métallique.

Il est connu que l'apposition d'une pièce composite comprenant un matériau absorbant et émettant des rayonnements infrarouges lointains contre la peau d'un individu lui produit des effets bénéfiques tels que décrit dans le document FR-A-2994393.

De manière non exhaustive, voici quelques effets bénéfiques :
- augmenter une sensation de chaleur au niveau de la peau,
- augmenter le flux sanguin au niveau de la peau grâce à la dilatation des vaisseaux capillaires, ce qui favorise la microcirculation sanguine,
- activer le métabolisme,
- activer la réponse musculaire et nerveuse,
- diminuer les sensations de douleur,
- améliorer le retour veineux,
- améliorer les tissus cicatriciels même anciens,
- améliorer le sommeil.

Pour que ces effets bénéfiques soient optimaux, il est important de maintenir le matériau absorbant et émettant des rayonnements infrarouges lointains au plus près de la peau d'un individu, aux emplacements déterminés choisis.

Le document FR-A-2994393 décrit la composition d'un patch incorporant des matériaux émettant des infrarouges lointains et propose de fixer le patch sur la peau à l'aide d'un sparadrap^{®} ou de tout autre moyen adapté de fixation sur la peau ou, alternativement d'incorporer le patch dans un vêtement, un drap, une couverture, un sac de couchage...

Le document FR-A-2897075 décrit comment associer un matériau émettant des infrarouges lointains directement avec une étoffe.

Lorsqu'un matériau émettant des infrarouges lointains est associé avec une étoffe, un vêtement ou équivalent, on ne garantit pas le maintien en place de la pièce incorporant le matériau émettant des infrarouges lointains. En effet, l'étoffe, le vêtement ou équivalent peut se froisser ou se déplacer par rapport au corps de l'individu, notamment lorsqu'il effectue un mouvement.

Lorsqu'un matériau émettant des infrarouges lointains est associé à une surface interne d'un patch fixé sur la peau grâce à un adhésif, cela permet de maintenir la position du patch sur le corps de l'individu. Cependant, la surface interne destinée à être en contact avec la peau ne présente pas de propriétés d'adhésion. En conséquence, cette surface peut se plisser, onduler ou se froisser ce qui peut produire une application non uniforme du patch sur la zone ciblée. D'autre part, s'il faut déplacer pour repositionner le patch, cette solution ne s'avère pas pratique car il faut préférentiellement utiliser d'autres adhésifs.

Le document FR-A-3046936 décrit un film multicouche de matériau composite comprenant une couche externe formée d'un polymère non collant sec au touché et d'une couche interne, destinée à être en contact avec la peau, formée d'une matrice à base de polymère auto-adhésif dans laquelle sont dispersées des particules de d'oxydes métalliques. Le mélange de particules d'oxydes métalliques avec une matrice à base de polymère auto-adhésif permet de garantir un contact continu entre la couche interne et la peau. La couche interne est directement fixée sur une couche externe en polymère. Dans ce document, la couche interne est définie par une épaisseur inférieure à 350 microns et la couche externe est définie par une épaisseur inférieure à 60 microns. L'épaisseur de la couche interne est ainsi limitée car si elle est trop importante, les propriétés d'adhésion de la couche peuvent être dégradées et il y aura un risque de flottement ou d'affaissement de la couche interne, ce qui peut ne pas être agréable.

WO 2015/171467 A1 décrit un article comprenant une composition qui comprend une biocéramique, qui, lorsqu'elle est chauffée ou exposée à la chaleur, fournit un effet biomodulateur ou physiologique lorsque l'article est appliqué à une personne, ladite biocéramique comprenant environ 20 % en poids à environ 80 % en poids de kaolinite (Al₂Si₂O₅(OH)₄); environ 1 % en poids à environ 30 % en poids de tourmaline; environ 1 % en poids à environ 40 % en poids d'oxyde d'aluminium (Al2O3); environ 1 % en poids à environ 40 % en poids de dioxyde de silicium (SiCh); et environ 1 % en poids à environ 20 % en poids d'oxyde de zirconium (ZrCb). La composition comprend en outre un substrat, un liant, un solvant, un polymère ou une encre; les solvants adaptés sont à sélectionner parmi les huiles grasses telles que l'huile de sésame, ou des esters d'acides gras synthétiques, tels que l'oléate d'éthyle ou des triglycérides, ou des liposomes.

Le but de l'invention est de proposer une construction d'une pièce composite comprenant un matériau émettant des infrarouges lointains améliorant les effets bénéfiques des oxydes métalliques.

Un but est notamment de proposer une couche d'oxyde métallique collante sur toute la surface interne de la pièce composite.

Un autre but est de répartir les oxydes métalliques sur une épaisseur significative de la pièce composite sans être au détriment de sa tenue.

Un autre but est de proposer une pièce composite confortable à porter contre la peau.

L'invention propose une pièce composite destinée à être en contact direct avec la peau d'un individu comprenant une base textile ayant une épaisseur délimitée par une face inférieure et une face supérieure.

La pièce composite est caractérisée par le fait qu'elle comprend une pâte collante composée d'un mélange d'une colle avec d'une pâte d'oxyde comprenant au moins un oxyde métallique, la pâte collante étant au moins partiellement incorporée dans la base textile et s'étendant, depuis la face inférieure de la base textile, pour former une couche interface d'épaisseur et au moins un composant hydrophile, et la pâte d'oxyde comprenant un solvant organique choisi parmi les solvants suivants: l'acétate d'éthyle, l'essence S, l'acétate de butyle et la méthyle éthyl cétone.

Grâce à cette construction, l'utilisateur est garanti du maintien en place de la pièce composite incorporant le matériau émettant des infrarouges lointains. D'autre part, du fait que la pâte collante étant au moins partiellement incorporée dans la base textile, cela permet d'accroitre la proportion d'oxydes métalliques dans l'épaisseur totale de la pièce composite. Or, il s'avère que ce maillage 3D permet une amélioration significative de l'action des oxydes métalliques sur la peau. La base textile sert d'armature qui permet d'améliorer la tenue de la couche comprenant des oxydes métalliques. Enfin, pour que les oxydes métalliques produisent leurs effets bénéfiques de manière optimum, il s'avère primordial de diffuser de l'eau à travers l'épaisseur de la pièce de recouvrement. Cela est rendu possible grâce à la présence d'au moins un composant hydrophile dans la pièce, celui-ci permettant d'amener l'eau en contact avec les oxydes métalliques ce qui active ses effets auprès des cellules de la peau sous-jacentes.

Selon des aspects avantageux mais non obligatoires de l'invention, une telle pièce composite peut incorporer une ou plusieurs des caractéristiques suivantes, prises dans toute combinaison techniquement admissible :
- Le composant hydrophile est la base textile et/ou un composant incorporé dans la pâte collante.
- La base textile est un textile tricoté, tissé ou non-tissé.
- La colle comprend au moins un élément parmi : le polyuréthane, l'acrylique, le copolymère polystyrène/butadiène (SBS), le latex naturel ou artificiel (polyisoprène), le gel d'élastomère de silicone, l'élastomère de silicone, le caoutchouc butyle, le caoutchouc naturel, le caoutchouc nitrile, le styrène bloc copolymère, le styrène.
- La pâte d'oxydes comprend au moins un oxyde métallique parmi : l'oxyde d'aluminium, l'oxyde de titane, l'oxyde de zirconium, l'oxyde d'Ytrium, l'oxyde de magnésium, l'oxyde de silicium, les oxydes de fer, l'oxyde de cobalt, l'oxyde de manganèse, l'oxyde de cuivre, l'oxyde de zinc, l'oxyde de chrome.
- La taille du au moins un oxyde métallique est supérieure à 2 micromètres et inférieure à 20 micromètres.
- La pâte d'oxyde présente une proportion du ou des solvant(s) organique(s) comprise entre 0,25% et 10% notamment entre 0,50% et 7,50%, et de préférence entre 1% et 5 % massique après séchage.
- Le séchage est réalisé à une température comprise entre 100°C et 200°C, notamment entre 115°C et 185°C, et de préférence entre 130 et 170°C pendant une durée comprise entre 30 secondes et 10 minutes, notamment entre 1 minute et 7 minutes et de préférence entre 2 minutes et 5 minutes.
- La pâte collante comprend également un composant hydrophile parmi les composants suivants: les carboxy-methyl-celluloses, le polyéthylène glycol.
- La pâte collante présente une proportion de composants hydrophile comprise entre 0,25% et 20% notamment entre 0,50% et 10%, et de préférence entre 1% et 5 % massique après séchage.
- La pâte collante comprend entre 50 et 85 % massique de colle 31 et entre 15 et 50 % massique de pâte d'oxydes.
- La pâte collante comprend entre 50 et 85 % de colle 31 et entre 15 et 50 % de pâte d'oxydes.
- La pièce composite comprend une couche de protection recouvrant, au moins partiellement, la face supérieure de la base textile.
- la pâte d'oxydes comprend un composant permettant d'organiser ses molécules de carbone en sphères et nanotubes afin d'augmenter les flux de protons et d'électrons entre les oxydes et l'eau.
- le composant permettant d'orienter ses molécules de carbone en sphère est une poudre de carbone riche en différents Fullerènes.
- le composant permettant d'orienter ses molécules de carbone en sphère est une poudre de Shungite.
- En d'autres termes, la pâte d'oxydes comprend une poudre de carbone riche en différents Fullerènes.
- Ainsi, grâce à cette disposition, la poudre de carbone riche en différents Fullerènes permet d'organiser ses molécules de carbone en sphères et nanotubes afin d'augmenter les flux de protons et d'électrons entre les oxydes et l'eau. En effet, poudre de carbone riche en différents Fullerènes peut s'organiser en nanotubes et peut agir comme un accélérateur de flux protoniques et électroniques, notamment au sein des organismes vivants.
- le composant présente une proportion de la poudre de Carbone riche en fullerènes comprise entre 1% et 80% notamment entre 2% et 67%, et de préférence entre 5 % et 50% massique de la pâte d'oxydes.

D'autre caractéristiques et avantages de l'invention seront mieux compris à l'aide de la description qui va suivre, en regard des dessins annexés illustrant, selon des formes de réalisation non limitatives, comment l'invention peut être réalisée, et dans lequel :
- la figure 1 est une vue en perspective d'une portion d'une pièce composite selon un premier mode de réalisation de l'invention ;
- la figure 2 est une vue en coupe selon le plan P de la figure 1 ;
- la figure 3 est une vue en coupe analogue à celle de la figure d'une portion d'une pièce composite selon un deuxième mode de réalisation de l'invention ;
- la figure 4 est un schéma représentant le processus d'obtention d'une pièce composite selon un premier mode de réalisation de l'invention.

L'usage de matériau émettant des infrarouges lointains contre la peau d'un utilisateur apporte de nombreux effets bénéfiques. Ceux-ci ont été largement décrits dans les documents de l'art antérieur cités précédemment : FR-A-2994393, FR-A-2897075 ou encore FR-A-3046936. Cela permet potentiellement l'amélioration d'au moins l'une des fonctions suivantes :
- confort accru en particulier contre les douleurs/raideurs péri-articulaires et musculaires ;
- cicatrisation;
- réduction du stress et de la fatigue musculaire ;
- soulagement des spasmes musculaires ;
- régulation de la circulation périphérique ;
- augmentation de la sensation de bien-être ;

Cependant, tous les documents de l'art antérieur restent silencieux sur plusieurs paramètres qui s'avèrent important pour l'efficacité de l'action des oxydes métalliques sur la peau, à savoir l'importance d'une répartition volumique des oxydes métalliques dans l'épaisseur de la pièce de recouvrement et l'importance de faire diffuser de l'eau vers les oxydes métalliques ce qui active fortement leurs effets auprès des cellules sous-jacentes (derme, capillaire, nerf, muscle, organe).

L'invention porte sur ces deux paramètres et propose notamment une construction particulière permettant d'accroitre la présence des oxydes métalliques sur une plus grande épaisseur de la pièce composite et de diffuser l'eau à travers la pièce composite.

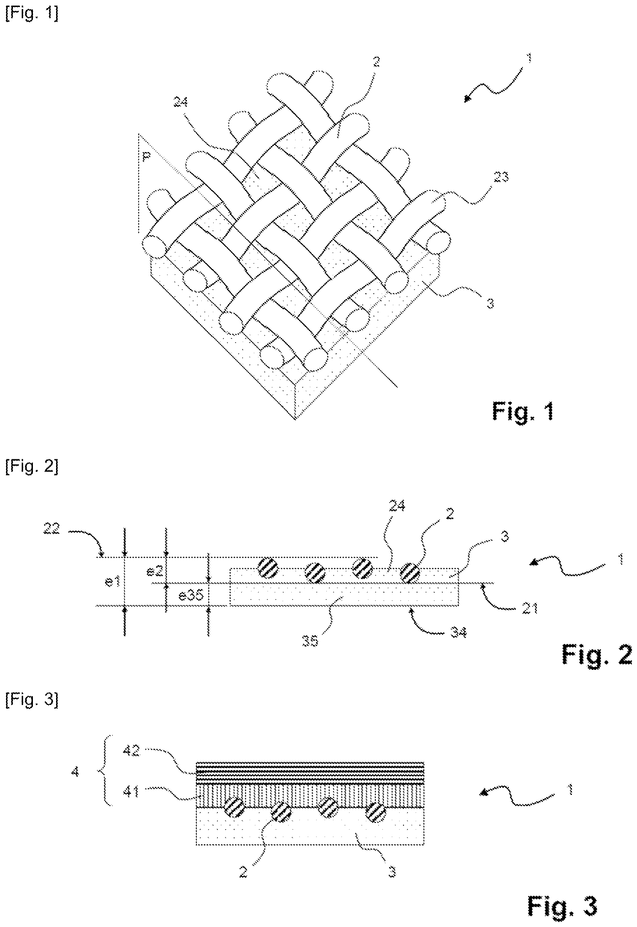

Selon l'invention et comme illustré aux figures 1 et 2, la pièce composite 1 comprend une base collante 3 au moins partiellement incorporée dans une base textile 2.

La base textile 2 peut être un tricot, un tissé ou un non-tissé. Dans l'exemple illustré, il s'agit d'un tissu formé d'un entrelacement de fibres 23 créant ainsi un réseau d'interstices 24. La base textile 2 est délimitée, dans son épaisseur e2, par une face inférieure 21 et une face supérieure 22.

Selon un mode de réalisation avantageux, la base textile 2 est hydrophile. Par exemple, les fibres de la base textile tissée, non tissée ou tricotée sont composées de matières naturelles type coton, lin, laine, et ou de matières artificielles type polyamides et/ou polyesters.

La base collante 3 est composée d'un mélange d'au moins une colle 31 et d'une pâte d'oxydes 32 comprenant au moins un oxyde métallique.

La colle 31 comprend au moins un élément parmi : le polyuréthane, l'acrylique, le copolymère polystyrène/butadiène (SBS), le latex naturel ou artificiel (polyisoprène), le gel d'élastomère de silicone, l'élastomère de silicone, le caoutchouc butyle, le caoutchouc naturel, le caoutchouc nitrile, le styrène bloc copolymère, le styrène.

La colle peut ainsi être une colle naturelle ou une colle de synthèse.

Selon un mode de réalisation, pour assurer un meilleur maintien en position, la colle utilisée est une colle mono-adhésive, c'est-à-dire, qu'elle ne permet pas un repositionnement de la pièce composite. Une fois mis en place, la pièce composite n'est pas prévue pour être retirée pour se recoller à un autre emplacement.

La pâte d'oxydes 32 comprend au moins un oxyde métallique parmi l'oxyde d'aluminium, l'oxyde de titane, l'oxyde de zirconium, l'oxyde d'Ytrium, l'oxyde de magnésium, l'oxyde de silicium, les oxydes de fer, l'oxyde de cobalt, l'oxyde de manganèse, l'oxyde de cuivre, l'oxyde de zinc, l'oxyde de chrome.

Pour obtenir une meilleure action des oxydes métalliques, la pâte d'oxydes comprend un mélange d'au moins trois oxydes métalliques, notamment parmi les oxydes métalliques décrits précédemment.

La taille des oxydes métalliques est déterminante pour agir sur le corps et ne pas gêner les mouvements de l'utilisateur. Aussi, leur taille est avantageusement supérieure à 2 micromètres et/ou inférieure à 20 micromètres.

La pâte d'oxydes 32 peut également comprendre d'autres composants que des oxydes métalliques. Elle comprend un solvant organique choisi parmi les solvants suivants l'acétate d'éthyle, l'essence S, l'acétate de butyle, la méthyle éthyle cétone.

Selon un mode de réalisation, la pâte d'oxyde présente une proportion du ou des solvant(s) organique(s) comprise entre 0,25% et 10% notamment entre 0,50% et 7,50%, et de préférence entre 1% et 5 % massique après séchage. Avantageusement, le solvant permet le bon mélange de la colle et de la pâte d'oxyde, au préalable avant séchage.

Après séchage, le solvant permet d'obtenir une bonne texture de colle et une bonne accroche de la colle malgré la charge importante d'oxydes minérales.

Selon un mode de réalisation, le séchage est réalisé à une température comprise entre 100°C et 200°C, notamment entre 115°C et 185°C, et de préférence entre 130 et 170°C pendant une durée comprise entre 30 secondes et 10 minutes, notamment entre 1 minute et 7 minutes et de préférence entre 2 minutes et 5 minutes.

Selon un mode de réalisation, la pâte collante 3 comprend un composant permettant d'organiser ses molécules de carbone en sphères et nanotubes afin d'augmenter les flux de protons et d'électrons entre les oxydes et l'eau. En effet, le composant permettant d'orienter ses molécules de carbone en sphère est une poudre à base de Shungite et/ou une poudre de carbone riche en différents Fullerènes.

En d'autres termes, la pâte d'oxydes comprend une poudre à base de Shungite et/ou une poudre de carbone riche en différents Fullerènes.

Le composant présente une proportion de la poudre de Carbone riche en fullerènes comprise entre 1% et 80% notamment entre 2% et 67%, et de préférence entre 5 % et 50% massique de la pâte d'oxydes.

Ainsi, la poudre à base de Shungite et/ou la poudre de carbone riche en différents Fullerènes permet d'organiser ses molécules de carbone en sphères et nanotubes afin d'augmenter les flux de protons et d'électrons entre les oxydes et l'eau. En effet, poudre de carbone riche en différents Fullerènes peut s'organiser en nanotubes et peut agir comme un accélérateur de flux protoniques et électroniques, notamment au sein des organismes vivants.

La pâte collante comprend également un composant hydrophile parmi les composants suivant : les carboxy-methyl-celluloses, le polyéthylène glycol. Ce ou ces composant(s) hydrophile représente une proportion comprise entre 0,25% et 20% massique notamment entre 0,50% et 10% massique, et de préférence entre 1% et 5% massique après séchage de la pâte collante.

La pâte collante comprend entre 50 et 85 % de colle 31 et entre 15 et 50 % de pâte d'oxydes et de préférence entre 50 et 85 % massique de colle 31 et entre 15 et 50 % massique de pâte d'oxydes.

La pièce composite comprend une couche de protection recouvrant, au moins partiellement, la face supérieure de la base textile.

Selon un mode de réalisation, la pâte collante 3 comprend entre 50 et 85 % de colle 31 et entre 15 et 50 % de pâte d'oxydes 32. Cette proportion permet d'avoir un bon équilibre entre l'adhésion sur la peau et l'action des oxydes métalliques sur le corps.

Selon un mode de réalisation, la pâte collante 3 comprend également un composant hydrophile 33. Par exemple, ce composant hydrophile est un composant parmi : les carboxy-methyl-celluloses, le polyéthylène glycol.

Selon l'invention, la pâte collante 3 est apposée sur la face inférieure 21 de la base textile 2. La pâte collante 3 définit une surface de contact 34 libre, destinée à être en contact avec la peau de l'utilisateur. A l'opposée, la pâte collante 3 s'infiltre dans la base textile 2 et notamment à travers les interstices 24. Ainsi, la pâte collante 3 est au moins partiellement incorporée dans la base textile 2 et s'étend, depuis la face inférieure 21 de la base textile, pour former une couche interface 35 d'épaisseur e35. Ainsi, les oxydes métalliques de la pâte collante sont répartis non seulement dans l'épaisseur e35 de la couche interface 35 mais également sur une partie de l'épaisseur e2 de la base textile 2 en se plaçant dans les interstices 24 de celle-ci. Cela permet donc d'augmenter la présence d'oxydes métalliques dans l'épaisseur et de la pièce composite 1.

Préférentiellement, la couche interface 35 comprend une épaisseur e35 comprise entre 50 microns et 300 microns et, de préférence, entre 50 microns et 150 microns. De même, la base textile 2 comprend préférentiellement une épaisseur e2 comprise entre 150 microns et 1000 microns et, de préférence, entre 200 microns et 500 microns.

Pour que les oxydes métalliques exercent pleinement leurs effets bénéfiques, il est important que ceux-ci soit à proximité d'eau et, plus particulièrement, de l'eau produit par la peau de l'utilisateur, comme par exemple, de la sueur. Aussi, pour améliorer l'efficacité du rayonnement des oxydes métalliques, il est préférable de diffuser l'eau présente au niveau de la surface de contact 34, à l'interface entre la peau et la pièce composite, à l'intérieur de la pièce composite. Pour capter cette eau présente à l'interface, l'invention propose d'intégrer des composants hydrophiles dans la pièce composite.

Selon un premier exemple, le composant hydrophile est la base textile 2 couplés à une pâte collante 3 comprenant des espaces pour le passage d'eau à travers la couche interface 35. Ainsi, la base textile va pomper l'eau interface pour la diffuser dans la pièce composite.

Selon un autre exemple, on ajoute des composants hydrophiles dans la pâte collante 3 ce qui permet de créer des vecteurs de diffusion de l'eau. L'eau est ainsi canalisée et peut pénétrer à travers la couche interface 35 grâce à ces composants hydrophile. Dans cet exemple, la base textile peut être également hydrophile, ce qui accentue encore la captation de l'eau à la surface de contact 34.

Selon un mode de réalisation illustré à la figure 3, la pièce composite 1 comprend également une couche de protection 4 s'étendant depuis la face supérieure 22 de la base textile 2. Dans cet exemple, la couche de protection 4 comprend un premier revêtement d'isolation 41 permettant de stopper la diffusion de la pâte collante 3. Ce revêtement d'isolation 41 est préférentiellement respirant. Il peut être réalisé par une couche tissée, tricotée, non tissée et une membrane. Cette couche d'isolation permet aussi d'éviter d'avoir une surface collante du côté de la face supérieure de la pièce composite, ce qui facilite la manipulation pour la mise en place de la pièce composite sur la peau. En complément, cette couche d'isolation 41 peut être recouverte par un revêtement de décoration 42 afin d'améliorer l'esthétisme de la pièce composite 1. Le revêtement de décoration 42 peut être réalisé par une couche tissée, tricotée, non tissée, membranée ou non.

Selon un mode de réalisation, la pièce composite est poreuse à l'eau. Ainsi, elle permet la circulation d'eau à travers son épaisseur. Cela augmente la respirabilité et les effets des oxydes métalliques, l'eau constituant alors un vecteur de transmission des rayonnements infrarouges lointains. Ainsi, on obtient une bonne transmission de l'énergie infrarouge vers les tissus vivants ce qui permet également une meilleure diffusion de ce rayonnement à travers le derme, d'où l'importance que la pièce soit bien plaquée contre la peau.

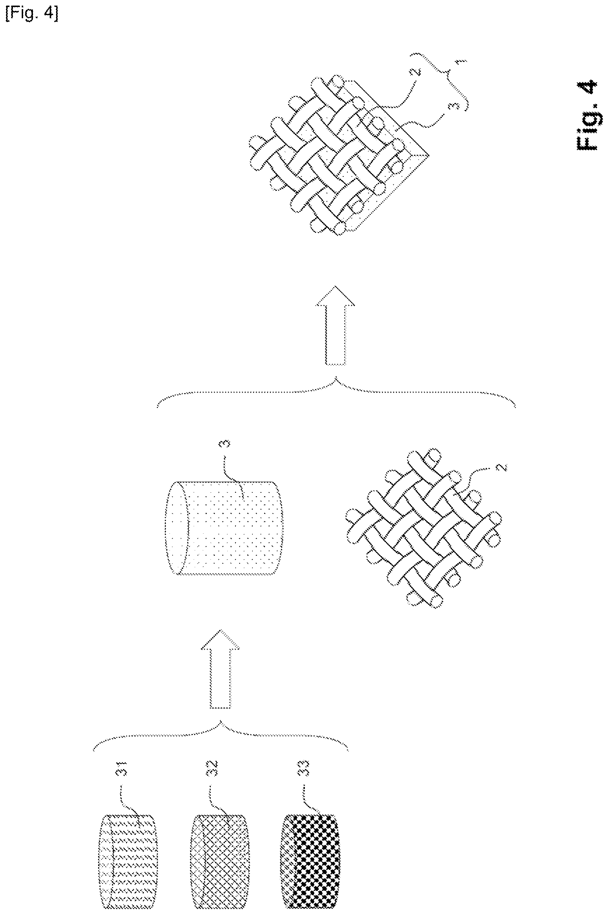

La figure 4 illustre un procédé pour réaliser la pièce composite 1 selon l'invention. Dans un premier temps, on réalise la pâte collante 3 en mélangeant une colle 31 avec une pâte d'oxydes métalliques 32. Selon un mode de réalisation, comme représenté dans ce schéma, on incorpore également un composant hydrophile 33 dans la pâte collante 3. Dans un deuxième temps, on applique la pâte collante 3 sur la face inférieure 21 de la base textile 2. On obtient alors la pièce composite 1.

Pour confirmer les effets bénéfiques de l'invention, plusieurs essais ont été réalisés.

Un premier essai avait pour but d'étudier l'impact d'un patch selon l'invention sur la souplesse.

L'essai a été réalisé avec un panel de 60 personnes (40 hommes/ 20 femmes) âgées de 21 à 71 ans. On leur a demandé de toucher le sol avec les mains par flexion antérieure du tronc. Tous les patients arrivant spontanément à toucher le sol, sont posés sur un promontoire leur permettant d'avoir les mains dans le vide, plus bas que leur plante des pieds.

Puis, chaque patient reçoit 8 patchs:
- un patch sur chaque pied face antérieure entre le 4e et le 5e métatarse,
- un patch de part et d'autre des vertèbres T11,
- un patch sur l'appendice xiphoïde,
- un patch sur chaque côté des fessiers,
- un patch sur les jambiers antérieurs.

Le port des patchs avant mesure est d'une demi-heure.

Ces patchs sont chargés à environs 35g d'oxydes métalliques par m2.

On constate alors une amélioration de la souplesse chez 88 % des patients, avec augmentation de 1 à 7 cm de la distance main sol.

Un deuxième essai avait pour but d'étudier l'impact d'un patch selon l'invention sur la cicatrisation.

Les patchs ont été utilisés plusieurs fois sur des cicatrices anciennes datant au minimum d'un an et jusqu'à plus de 30 ans, et dont l'évolution était figée et stoppée. Les cicatrices traitées étaient des cicatrices chéloïdes, boursouflées, adhérentes, larges, inesthétiques, douloureuses au toucher, etc.

Le traitement consistait à une pose de patch constante sur toute la longueur de la cicatrice pendant 1 à 3 mois en fonction de la gravité des cicatrices.

L'étude en interne a été réalisée sur 30 patients en kinésithérapie tout au long de l'année 2017.

Les traitements ont montré une amélioration systématique des cicatrices plus ou moins prononcée tant sur le plan esthétique que sur les inconforts associés à ces cicatrices tel que les adhérences ou les douleurs associées.

Chez les 9 personnes qui avaient des douleurs au niveau de leurs cicatrices, les sensations somesthésiques des patients étaient améliorées avec plus de sensibilité fine au toucher et des douleurs estompées voir complètement disparues pour deux d'entre eux.

Chez les 12 personnes qui avaient des cicatrices chéloïdes, boursouflées, larges et donc inesthétiques, un aplanissement significatif et une diminution de la surface cicatricielle a été constaté ainsi qu'une atténuation de l'aspect inflammatoire rougeâtre de la cicatrice. A noter que ce type de cicatrice nécessite une durée de traitement plus longue d'au moins 3 mois.

Chez les 9 personnes qui avaient des cicatrices adhérentes, la peau cicatricielle retrouve une souplesse physiologique se rapprochant de la peau saine environnante, leur permettant une amélioration du mouvement de la zone cicatricielle.

En complément, il a été constaté que l'amélioration des cicatrices via un traitement avec les patchs entraînait la disparition de gênes et de douleurs associées aux cicatrices perturbées.

En conclusion, les patchs contribuent à retrouver une peau saine et physiologique sur les tissus cicatriciels quel que soit l'âge de la cicatrice. Les cicatrices ont retrouvé des aspects plus physiologiques, notamment pour les cicatrices chéloïdes. De plus, les résultats laissent à penser que des cicatrices problématiques, même très anciennes peuvent être à l'origine de douleurs associées et c'est pourquoi il est important de traiter toutes les cicatrices.

Un troisième essai avait pour but d'étudier l'impact d'un patch selon l'invention sur les douleurs arthrosiques.

Les patchs ont été utilisé sur des personnes présentant des douleurs arthrosiques que ce soit au niveau des cervicales, des genoux, des hanches ou des lombaires. L'arthrose provoque très souvent des raideurs, des douleurs et des difficultés dans le mouvement de l'articulation touchée. Elle est accompagnée de phases aigües où la douleur augmente et de phases latentes caractérisées par les raideurs et des difficultés dans le mouvement.

Les 40 personnes, âgées entre 44 et 70 ans, ont eu le droit à un protocole défini en fonction de la localisation de l'arthrose. Le principe de base étant d'entourer l'articulation douloureuse avec au moins deux patchs

Chez les 40 personnes, quel que soit la zone arthrosique traitée, il a été constaté que le mouvement était facilité et que les raideurs étaient diminuées.

Chez les 13 personnes atteintes d'arthrose du genou et traitées avec ces patchs, la marche était plus fluide et moins douloureuse. Les phases aigües étaient aussi moins douloureuses que sans les patchs.

Les 10 personnes atteintes d'arthrose de hanche ainsi que les 10 personnes atteintes d'arthrose cervicale et les 7 personnes atteintes d'arthrose lombaire ont aussi senti une libération dans les mouvements avec une amplitude augmentée et des douleurs moins présentes. Pour les personnes atteintes d'arthrose de hanche, la marche et la position assise étaient plus confortables. Pour les 10 personnes atteintes d'arthrose cervicale, les raideurs dans le cou, notamment au réveil, étaient moins importantes et les mouvements de rotations du coup étaient facilités. Enfin pour les 7 personnes atteintes d'arthrose lombaire, les douleurs au réveil et lors de la flexion du dos étaient diminuées avec une augmentation dans l'amplitude de flexion.

Le traitement de l'arthrose à l'aide de ces patchs amène plus de confort aux patients qui constatent une amélioration au quotidien de leurs mouvements. Quelle que ce soit la zone traitée, les sujets ressentent donc tous les mêmes effets c'est-à-dire moins de raideurs, plus d'amplitudes et moins de douleurs. Les patchs semblent donc être une solution efficace dans le traitement quotidien de l'arthrose, quels que soient l'âge du patient ou la gravité de l'arthrose.

L'invention n'est pas limitée aux modes de réalisation précédemment décrits. Il est également possible de combiner ces modes de réalisation. L'invention s'étend à tous les modes de réalisation couverts par les revendications.

### Nomenclature

1- Pièce de recouvrement
2- Base textile
   21- Face inférieure
   22- Face supérieure
   23- Fibre
   24- interstice
3- Pate collante
   31- Colle
   32- Pate d'oxydes métalliques
   33- Composant hydrophile
   34- Surface de contact
   35- Couche interface
4- Couche de protection
   41- Revêtement d'isolation
   42- Revêtement de décoration

## Revendications

1. Pièce composite (1) destinée à être en contact direct avec la peau d'un individu comprenant une base textile (2) ayant une épaisseur (e2) délimitée par une face inférieure (21) et une face supérieure (22)
**caractérisée en ce qu'**elle comprend
- une pâte collante (3) composée d'un mélange d'une colle (31) avec une pâte d'oxydes (32) comprenant au moins un oxyde métallique, la pâte collante étant au moins partiellement incorporée dans la base textile et s'étendant, depuis la face inférieure de la base textile, pour former une couche interface (35) d'épaisseur (e35), et
- au moins un composant hydrophile (33) ;
la pâte d'oxyde (32) comprenant un solvant organique choisi parmi les solvants suivants l'acétate d'éthyle, l'essence S, l'acétate de butyle, la méthyle éthyle cétone.

2. Pièce composite (1) selon la revendication 1 **caractérisée en ce que** le composant hydrophile est la base textile et/ou un composant incorporé dans la pâte collante.

3. Pièce composite (1) selon l'une des revendications précédentes **caractérisée en ce que** la base textile est un textile tricoté, tissé ou non-tissé.

4. Pièce composite (1) selon l'une des revendications précédentes **caractérisée en ce que** la colle (31) comprend au moins un élément parmi : le polyuréthane, l'acrylique, le copolymère polystyrène/butadiène (SBS), le latex naturel ou artificiel (polyisoprène), le gel d'élastomère de silicone, l'élastomère de silicone, le caoutchouc butyle, le caoutchouc naturel, le caoutchouc nitrile, le styrène bloc copolymère, le styrène.

5. Pièce composite (1) selon l'une des revendications précédentes **caractérisée en ce que** la pâte d'oxydes (32) comprend au moins un oxyde métallique parmi : l'oxyde d'aluminium, l'oxyde de titane, l'oxyde de zirconium, l'oxyde d'Ytrium, l'oxyde de magnésium, l'oxyde de silicium, les oxydes de fer, l'oxyde de cobalt, l'oxyde de manganèse, l'oxyde de cuivre, l'oxyde de zinc, l'oxyde de chrome.

6. Pièce composite (1) selon l'une des revendications précédentes **caractérisée en ce que** la taille du au moins un oxyde métallique est supérieure à 2 micromètres et inférieure à 20 micromètres.

7. Pièce composite (1) selon l'une des revendications précédentes **caractérisée en ce que** la pâte collante (3) comprend également un composant hydrophile (33) parmi les composants suivant : les carboxy-methyl-celluloses, le polyéthylène glycol.

8. Pièce composite (1) selon l'une des revendications précédentes **caractérisée en ce que** la pâte collante (3) comprend entre 50 et 85 % de colle 31 et entre 15 et 50 % de pâte d'oxydes.

9. Pièce composite (1) selon l'une des revendications précédentes **caractérisée en ce qu'**elle comprend une couche de protection (4) recouvrant, au moins partiellement, la face supérieure (22) de la base textile.

10. Pièce composite (1) selon l'une des revendications précédentes, **caractérisée en ce que** la pâte d'oxydes comprend un composant permettant d'organiser ses molécules de carbone en sphères et nanotubes afin d'augmenter les flux de protons et d'électrons entre les oxydes et l'eau.

11. Pièce composite (1) selon la revendication précédente, **caractérisée en ce que** le composant permettant d'orienter ses molécules de carbone en sphère est une poudre de carbone riche en différents Fullerènes.

12. Pièce composite (1) selon l'une des revendications précédentes, **caractérisée en ce que** la pâte d'oxydes comprend une poudre de carbone riche en différents Fullerènes et de préférence la poudre de Carbone riche en fullerènes représente en proportion de 5 à 50% de la pâte d'oxydes.

13. Pièce composite (1) selon l'une des revendications précédentes, **caractérisée en ce que** la pâte collante (3) définit une surface de contact libre (34) destinée à être en contact directement avec la peau de l'utilisateur

14. Pièce composite (1) selon l'une des revendications précédentes, **caractérisée en ce que** l'au moins un composant hydrophile (33), notamment lié à la base textile diffusant l'eau présente au niveau de la surface de contact 34, à l'interface entre la peau et la pièce composite, à l'intérieur de la pièce composite afin d'avoir des flux protoniques aqueux entre la peau et la pièce composite. ;

## Patentansprüche

1. Verbundteil (1), das dazu bestimmt ist, sich in direktem Kontakt mit der Haut eines Individuums zu befinden, umfassend eine textile Basis (2) mit einer Dicke (e2), die von einer Unterseite (21) und einer Oberseite (22) begrenzt wird,
**dadurch gekennzeichnet, dass** es umfasst
- eine Klebepaste (3), die aus einer Mischung aus einem Kleber (31) mit einer Oxidpaste (32) besteht, welche mindestens ein Metalloxid umfasst, wobei die Klebepaste mindestens teilweise in die textile Basis eingebettet ist und sich von der Unterseite der textilen Basis erstreckt, um eine Grenzflächenschicht (35) der Dicke (e35) zu bilden, und
- mindestens eine hydrophile Komponente (33);
wobei die Oxidpaste (32) ein organisches Lösungsmittel umfasst, ausgewählt aus den folgenden Lösungsmitteln: Ethylacetat, S-Benzin, Butylacetat, Methylethylketon.

2. Verbundteil (1) nach Anspruch 1, **dadurch gekennzeichnet, dass** es sich bei der hydrophilen Komponente um die textile Basis und/oder eine Komponente handelt, die in die Klebepaste eingebettet ist.

3. Verbundteil (1) nach einem der vorstehenden Ansprüche, **dadurch gekennzeichnet, dass** es sich bei der textilen Basis um ein gestricktes, gewebtes oder Vliestextil handelt.

4. Verbundteil (1) nach einem der vorstehenden Ansprüche, **dadurch gekennzeichnet, dass** der Kleber (31) mindestens ein Element umfasst aus: Polyurethan, Acryl, Polystyrol/Butadien-Copolymer (SBS), Natur- oder Kunstlatex (Polyisopren), Silikonelastomergel, Silikonelastomer, Butylkautschuk, Naturkautschuk, Nitrilkautschuk, Styrol-Blockcopolymer, Styrol.

5. Verbundteil (1) nach einem der vorstehenden Ansprüche, **dadurch gekennzeichnet, dass** die Oxidpaste (32) mindestens ein Metalloxid umfasst aus: Aluminiumoxid, Titanoxid, Zirkoniumoxid, Yttriumoxid, Magnesiumoxid, Siliziumoxid, Eisenoxiden, Kobaltoxid, Manganoxid, Kupferoxid, Zinkoxid, Chromoxid.

6. Verbundteil (1) nach einem der vorstehenden Ansprüche, **dadurch gekennzeichnet, dass** die Größe des mindestens einen Metalloxids größer als 2 Mikrometer und kleiner als 20 Mikrometer ist.

7. Verbundteil (1) nach einem der vorstehenden Ansprüche, **dadurch gekennzeichnet, dass** die Klebepaste (3) ebenfalls eine hydrophile Komponente (33) aus den folgenden Komponenten umfasst: Carboxymethylcellulosen, Polyethylenglykol.

8. Verbundteil (1) nach einem der vorstehenden Ansprüche, **dadurch gekennzeichnet, dass** die Klebepaste (3) zwischen 50 und 85 % Kleber (31) und zwischen 15 und 50 % Oxidpaste umfasst.

9. Verbundteil (1) nach einem der vorstehenden Ansprüche, **dadurch gekennzeichnet, dass** es eine Schutzschicht (4) umfasst, die die Oberseite (22) der textilen Basis mindestens teilweise bedeckt.

10. Verbundteil (1) nach einem der vorstehenden Ansprüche, **dadurch gekennzeichnet, dass** die Oxidpaste eine Komponente umfasst, die es ermöglicht, ihre Kohlenstoffmoleküle als Kugeln und Nanoröhren zu organisieren, um den Fluss von Protonen und Elektronen zwischen den Oxiden und Wasser zu erhöhen.

11. Verbundteil (1) nach dem vorstehenden Anspruch, **dadurch gekennzeichnet, dass** es sich bei der Komponente, die es ermöglicht, ihre Kohlenstoffmoleküle als Kugeln auszurichten, um ein Kohlenstoffpulver handelt, das reich an verschiedenen Fullerenen ist.

12. Verbundteil (1) nach einem der vorstehenden Ansprüche, **dadurch gekennzeichnet, dass** die Oxidpaste ein Kohlenstoffpulver umfasst, das reich an verschiedenen Fullerenen ist, und vorzugsweise das Fulleren-reiche Kohlenstoffpulver anteilsmäßig 5 bis 50 % der Oxidpaste ausmacht.

13. Verbundteil (1) nach einem der vorstehenden Ansprüche, **dadurch gekennzeichnet, dass** die Klebepaste (3) eine freie Kontaktfläche (34) definiert, die dazu bestimmt ist, sich in direktem Kontakt mit der Haut des Benutzers zu befinden.

14. Verbundteil (1) nach einem der vorstehenden Ansprüche, **dadurch gekennzeichnet, dass** die mindestens eine hydrophile Komponente (33) insbesondere in Verbindung mit der textilen Basis das Wasser, das im Bereich der Kontaktfläche (34), an der Grenzfläche zwischen der Haut und dem Verbundteil, im Inneren des Verbundteils vorhanden ist, verteilt, um über wässrige Protonenflüsse zwischen der Haut und dem Verbundteil zu verfügen.

## Claims

1. A composite part (1) intended to be in direct contact with the skin of an individual comprising a textile base (2) having a thickness (e2) delimited by a lower face (21) and an upper face (22)
**characterized in that** it comprises
- a sticky paste (3) composed of a mixture of an adhesive (31) with an oxide paste (32) comprising at least one metal oxide, the sticky paste being at least partially incorporated into the textile base and extending, from the lower face of the textile base, to form an interface layer (35) of a thickness (e35), and
- at least one hydrophilic component (33);
the oxide paste (32) comprising an organic solvent 2 selected from the following solvents: ethyl acetate, gasoline S, butyl acetate, methyl ethyl ketone.

2. The composite part (1) according to claim 1, **characterized in that** the hydrophilic component is the textile base and/or a component incorporated in the sticky paste.

3. The composite part (1) according to any of the preceding claims, **characterized in that** the textile base is a knitted, woven or non-woven textile.

4. The composite part (1) according to any of the preceding claims, **characterized in that** the adhesive (31) comprises at least one element among: polyurethane, acrylic, polystyrene/butadiene (SBS) copolymer, natural or artificial (polyisoprene) latex, silicone elastomer gel, silicone elastomer, butyl rubber, natural rubber, nitrile rubber, styrene block copolymer, styrene.

5. The composite part (1) according to any of the preceding claims, **characterized in that** the oxide paste (32) comprises at least one metal oxide among: aluminum oxide, titanium oxide, zirconium oxide, ytrium oxide, magnesium oxide, silicon oxide, iron oxides, cobalt oxide, manganese oxide, copper oxide, zinc oxide, chromium oxide.

6. The composite part (1) according to any of the preceding claims, **characterized in that** the size of the at least one metal oxide is greater than 2 micrometers and less than 20 micrometers.

7. The composite part (1) according to any of the preceding claims, **characterized in that** the sticky paste (3) also comprises a hydrophilic component (33) among the following components: carboxy-methyl-celluloses, polyethylene glycol.

8. The composite part (1) according to any of the preceding claims, **characterized in that** the sticky paste (3) comprises between 50 and 85% adhesive 31 and between 15 and 50% oxide paste.

9. The composite part (1) according to any of the preceding claims **characterized in that** it comprises a protective layer (4) covering, at least partially, the upper face (22) of the textile base.

10. The composite part (1) according to any of the preceding claims, **characterized in that** the oxide paste comprises a component making it possible to organize its carbon molecules into spheres and nanotubes in order to increase the fluxes of protons and of electrons between oxides and water.

11. The composite part (1) according to the preceding claim, **characterized in that** the component making it possible to orient its carbon molecules in a sphere is a carbon powder rich in various fullerenes.

12. The composite part (1) according to any of the preceding claims, **characterized in that** the oxide paste comprises a carbon powder rich in different fullerenes and preferably the carbon powder rich in fullerenes represents in a proportion from 5 to 50% of the oxide paste.

13. The composite part (1) according to any of the preceding claims, **characterized in that** the sticky paste (3) defines a free contact surface (34) intended to be in direct contact with the skin of the user

14. The composite part (1) according to any of the preceding claims, **characterized in that** the at least one hydrophilic component (33), in particular bonded to the water-diffusing textile base present at the contact surface 34, at the interface between the skin and the composite part, inside the composite part in order to have aqueous proton fluxes between the skin and the composite part.
